(19) Europäisches Patentamt
European Patent Office
Office européen des brevets

(11) Veröffentlichungsnummer: **0 001 217**
**A2**

(12) **EUROPÄISCHE PATENTANMELDUNG**

(21) Anmeldenummer: 78100729.9

(22) Anmeldetag: 23.08.78

(51) Int. Cl.[2]: C 07 D 331/02
C 07 C 149/18, C 07 C 149/24
C 07 C 149/06, C 07 C 149/20
C 10 M 1/38

(30) Priorität: 31.08.77 CH 10609/77

(43) Veröffentlichungstag der Anmeldung: 04.04.79 Patentblatt 79/7

(84) Benannte Vertragsstaaten: BE CH DE FR GB NL SE

(71) Anmelder: CIBA-GEIGY AG
Patentabteilung Postfach
CH-4002 Basel(CH)

(72) Erfinder: Michaelis, Peter, Dr.
Am Hangweg 8
D-6145 Lindenfels/Odenwald(DE)

(54) **Epithioverbindungen oder Mercaptone, und deren Verwendung als Schmiermittelzusätze.**

(57) Epithioverbindungen oder Mercaptane der Formel I

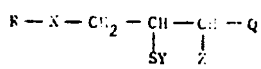
(I),

worin
X ein Sauerstoff- oder Schwefelatom, die Gruppen $-CH_2-$, $-CH(R_1)-$, $-COO-$, $-NH-$ oder $-NR_1-$ ist, Y und Z jeweils Wasserstoff oder zusammen eine direkte Bindung, Q, wen Y und Z zusammen eine direkte Bindung darstellen, Wasserstoff ist oder Q die Gruppen $-OH$, $-SH$, $-OR_1$, $-SR_1$, $-OOCR_1$, $-S-(CH_2)-_nOH$, $-S-(CH_2)-_nCOOR_1$, $-NHR_1$ oder $-N(R_1)_2$, n die Zahlen 1 bis 10, R und $R_1$ jeweils unabhängig voneinander einen gegebenenfalls durch Sauerstoff oder Schwefel unterbrochenen cyclischen oder acyclischen Kohlenwasserstoffrest aliphatischen oder aromatischen Charakters mit 4-30 Kohlenstoffatomen bedeuten mit der Bedingung, dass die Summe der C-Atome in den Gruppen R und $R_1$ 8-30 Kohlenstoffatome beträgt, eignen sich ausgezeichnet als Schmiermittelzusätze, indbesondere als "Extreme-Pressure"- und "Antiwear"- Additive.

EP 0 001 217 A2

3-11313/CGM 183/+

BEZEICHNUNG GEÄNDERT
siehe Titelseite

Schmiermittelzusätze

Die vorliegende Erfindung betrifft β-substituierte Epithioverbindungen und deren Anlagerungsprodukte, sowie deren Verwendung als Schmiermittelzusätze, insbesondere als "Extreme-Pressure"- und "Antiwear"-Additive.

Zugleich Schwefel und Phosphor enthaltende Verbindungen, wie schwefelhaltige Phosphorsäure- und Phosphorigsäureester und deren Verwendung als Schmiermittelzusätze sind schon seit langem bekannt, vgl. z.B. FR-PS 1 137 298 und Houben Weyl, Methoden der organischen Chemie, Thieme Verlag, Bd. 12/2, Seiten 93-95 und 742-748.

Unter den bekannten S-haltigen Estern der Phosphorsäuren wurden Vertreter entwickelt, die wirksame EP/AW-Additive für Schmiermittel darstellen. Diese Ester sind jedoch nicht ohne Nachteile. Z.B. zeigt Triphenylthionophosphat in vielen Fällen eine nur unzureichende Wirksamkeit. Die Trithiophosphite, besonders die in der US-PS 3,374,291 beschriebenen Trithiophosphite aus $PCl_3$ und SH-haltigen Carbonsäureestern, wirken besonders bei höheren Temperaturen auf Metalloberflächen korrosiv. Die in der US-PS 3,705,216 vorgeschlagenen Trithio- und Tetrathiophosphorsäurealkylester weisen ebenfalls

oft unzureichende Wirksamkeiten auf. Bei den gut wirksamen Zink-dialkyldithiophosphaten wird es als Nachteil empfunden, dass sie nicht rückstandslos verbrennen und daher auf Grund des Metallgehaltes und der relativ niedrigen Zersetzungstemperaturen z.B. beim Kontakt mit heissen Metalloberflächen zur Bildung von Ablagerungen neigen.

Es bestand deshalb das Bedürfnis nach phosphor- und metallfreien, sowie bei der Verbrennung keine Rückstände bildenden Oeladditiven. So werden beispielsweise in Chemical Abstracts 79, 1973, 94496x und 82, 1975, 88387h N-substituierte, Epithiogruppen enthaltende Fettsäureamide, wie beispielsweise N,N-Dibutyl-9,10-epithiostearamid oder N,N-Dibutyl-9,10,12,13-diepithiostearamid als gute EP/AW-Additive beschrieben. In der Zeitschrift Tribology, 3 (1970), 145, werden Epithiogruppen enthaltende Fettsäureester und Terpene mit ähnlichen Schmiermitteleigenschaften erwähnt. Ein übliches Herstellungsverfahren für solche Verbindungen ist die Schwefeladdition an die Doppelbindung von olefinischen Fettsäurederivaten. Mit dieser Reaktion werden die gewünschten Epithioverbindungen meistens nur in geringen Ausbeuten erhalten (vgl. Chem. Rev. 66 (1966), 297).

In der gleichen Publikation Chem. Rev. 66 (1966), 297 sind Niederalkyl- und Niederalkyläther-substituierte Epithioverbindungen beschrieben, die von den entsprechenden Epoxiden durch Umsetzung mit KSCN mit guten Ausbeuten erhalten werden.

Es wurde nun gefunden, dass mit Aryl- oder langkettigem Alkyläther, -Thioäther oder -Amin β-substituierte Epithioverbindungen und Umwandlungsprodukte mit protonischen Nucleophilen überraschenderweise sehr wirkungsvolle Schmiermittelzusätze, insbesondere EP/AW-Additive darstellen, die gegenüber anderen P-freien, allein S enthaltenden Produkten überlegen sind.

Gegenstand vorliegender Erfindung sind dementsprechend Epithioverbindungen oder Mercaptane der Formel I

$$R-X-CH_2-\underset{SY}{\underset{|}{CH}}-\underset{Z}{\underset{|}{CH}}-Q \qquad (I),$$

worin

X ein Sauerstoff- oder Schwefelatom, die Gruppen $-CH_2-$, $-CH(R_1)-$, $-COO-$, $-NH-$ oder $-NR_1-$ ist, Y und Z jeweils Wasserstoff oder zusammen eine direkte Bindung, Q, wenn Y und Z zusammen eine direkte Bindung darstellen Wasserstoff ist oder Q die Gruppen $-OH$, $-SH$, $-OR_1$, $-SR_1$, $-OOCR_1$, $-S-(CH_2)_n-OH$, $-S-(CH_2)_n-COOR_1$, $-NHR_1$ oder $-N(R_1)_2$, n die Zahlen 1 bis 10, R und $R_1$ jeweils unabhängig voneinander einen gegebenenfalls durch Sauerstoff oder Schwefel unterbrochenen cyclischen oder acyclischen Kohlenwasserstoffrest aliphatischen oder aromarischen Charakters mit 4-30 Kohlenstoffatomen bedeuten, mit der Massgabe, dass die Summe der C-Atome in den Gruppen R und $R_1$ 8-30 Kohlenstoffatome beträgt.

R und $R_1$ in ihrer Bedeutung als aliphatischer Kohlenwasserstoffrest können gegebenenfalls durch O- oder S-Atome unterbrochenes lineares, insbesondere aber verzweigtes Alkyl mit 4-30 Kohlenstoffatomen oder gegebenenfalls mit 1 oder 2 Alkylgruppen mit bevorzugt 1-12 Kohlenstoffatomen substituiertes Cycloalkyl oder Cycloalkylalkyl sein. In ihrer Bedeutung als aromatischer Kohlenstoffrest können R und $R_1$ gegebenenfalls durch 1 oder 2 Alkylgruppen mit bevorzugt 1-12 Kohlenstoffatomen substituiertes Aryl oder Aralkyl sein. Das Alkyl enthält hierbei bevorzugt 4-22, besonders aber 8-22 und insbesondere 8-18 Kohlenstoffatome und das Cycloalkyl 5-8 Ringkohlenstoffatome und ist bevorzugt Cyclohexyl. Aryl steht bevorzugt für Phenyl und Aralkyl für Benzyl, wobei das Cycloalkyl, Phenyl oder Benzyl bevorzugt mit 1 oder 2 Alkylgruppen substituiert ist.

Insbesondere sind R und $R_1$ in der Formel I verzweigtes Alkyl mit 8-18 Kohlenstoffatomen.

Beispiele für R sind n-Butyl, Isobutyl, tert.-Butyl, Pentyl, Hexyl, 2-Methylpentyl, Heptyl, Octyl, 2-Aethylhexyl, Nonyl, Decyl, Undecyl, Dodecyl, 2-Aethyldecyl, Tetradecyl, Octadecyl, Eicosyl, Docosyl, Tetracosyl und deren Isomerengemische, Methoxy-n-propyl, Octoxyäthyl, Octylthioäthyl, Cyclopentyl, Methylcyclopentyl, Aethylcyclopentylmethyl, Cyclohexylmethyl, Methylcyclohexyl, Methylphenyl, Aethylphenyl, tert.-Butylphenyl, Dimethylphenyl, Hexylphenyl, 2-Aethylhexylphenyl, Dinonylphenyl, Dodecylphenyl, Methylbenzyl, Nonylbenzyl, Dodecylbenzyl.

Bei den oben erwähnten Isomerengemischen handelt es sich um solche, die sich von über die Oxo-Synthese hergestellten und im Handel befindlichen Alkoholen ableiten. Diese, auch Oxo-Alkohole genannten Produkte, bestehen meistens aus einem Gemisch verzweigtkettiger, insbesondere primärer Alkohole.

Besonders interessant sind Epithioverbindungen oder Mercaptane der Formel I, worin X ein Sauerstoff- oder Schwefelatom oder die Gruppe -COO- ist, Y und Z die oben angegebene Bedeutung haben, Q, wenn Y und Z zusammen eine direkte Bindung darstellen, Wasserstoff ist oder Q die Gruppen -SH, $-SR_1$, $-S-(CH_2)_2-OH$, $-S-CH_2-COOR_1$, $-NHR_1$ bedeutet und R und $R_1$ jeweils unabhängig voneinander verzweigtes oder unverzweigtes $C_4$-$C_{22}$ Alkyl sind, mit der Massgabe, dass die Summe der C-Atome in den Gruppen R und $R_1$ 8-30 Kohlenstoffatome beträgt.

Von ganz besonderem Interesse sind Epithioverbindungen oder Mercaptane der Formel I, worin X ein Sauerstoffatom oder die Gruppe -COO- ist, Y und Z die oben angegebene Bedeutung haben, Q, wenn Y und Z zusammen eine direkte Bindung darstellen, Wasserstoff ist oder Q die Gruppen -SH, $-SR_1$, $-S-CH_2-COOR_1$ und $-NHR_1$ ist und R und $R_1$ jeweils unabhängig voneinander verzweigtes $C_8$-$C_{22}$ Alkyl bedeuten, mit der Massgabe, dass die Summe der C-Atome in den Gruppen R und $R_1$ 8-20 Kohlenstoffatome beträgt.

Bevorzugt sind Epithioverbindungen der Formel II,

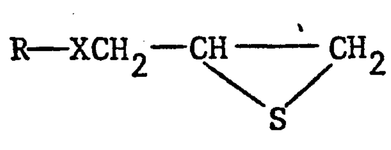

(II),

worin R verzweigtes $C_8$-$C_{18}$ Alkyl und X ein Sauerstoffatom oder die Gruppe -COO- bedeuten, sowie Mercaptane der Formel III

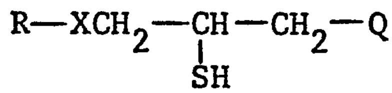 (III),

worin X die oben angegebene Bedeutung hat, Q die Gruppen $-SR_1$ oder $-S-CH_2-COOR_1$ bedeuten und R und $R_1$ verzweigtes $C_8$-$C_{18}$ Alkyl sind, mit der Massgabe, dass die Summe der C-Atome in den Gruppen R und $R_1$ 8-20 Kohlenstoffatome beträgt.

Die erfindungsgemässen Epithioverbindungen werden nach bekannten Methoden aus allgemein bekannten Epoxiden der Formel IV

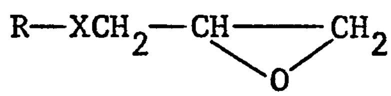 (IV),

worin R und X die oben angegebene Bedeutung haben, durch Umsetzung mit Alkali- bzw. Erdalkalithiocyanat oder Thioharnstoff, gewonnen. Eine entsprechende Herstellungsmethode ist z.B. in Org. Synth., 32 (1952), 39 beschrieben. Obengenannte Epoxide können nach bekannten Methoden z.B. durch Umsetzung von Mercaptanen mit Epichlorhydrin, gegebenenfalls in Anwesenheit geeigneter Katalysatoren hergestellt werden.

Zur Durchführung der Reaktion mit Thioharnstoff suspendiert man die berechnete Menge in der äquivalenten Menge etwa 10 %iger Schwefelsäure und tropft das in Dioxan gelöste Epoxyd unter gutem Rühren bei ca. 0°C - 5°C zu. Man erwärmt nach erfolgtem Zutropfen kurzzeitig auf 40°C und lässt dann eine ca. 20 %ige wässrige äquivalente Natriumcarbonatlösung unter starkem Rühren zutropfen. Beim Erwärmen auf ca. 50°C wird ein eventuell entstandener Niederschlag sofort wieder gelöst.

Zur Isolierung der Episulfide säuert man mit verd. Schwefelsäure an und extrahiert mehrmals mit Aether und wäscht neutral. Aus der ätherischen Lösung lassen sich nach Aufarbeitung die Episulfide in über 90 %iger Ausbeute isolieren.

Bei der weiteren Umsetzung der so erhaltenen Epithioverbindungen der Formel V

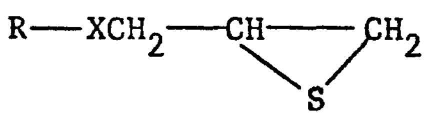 (V)

mit bekannten protonischen Nucleophilen der Formel VII

$$H - Q \qquad (VII),$$

wie beispielsweise Alkylmercaptane, Thioglycolsäureestern oder primären oder sekundären Alkylaminen gelangt man zu den erfindungsgemässen Mercaptanen der Formel VI

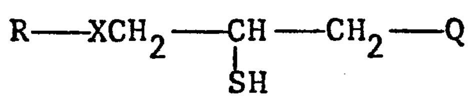 (VI),

wobei in den Formeln V, VI und VII R, Q und X die oben angegebene Bedeutung haben. Die Addition von protonischen Nucleophilen an Epithioverbindungen ist beispielsweise in JACS 69 (1947), 2675 beschrieben. Sie wird im allgemeinen bei Temperaturen zwischen 50 und 150°C, bevorzugt zwischen 70 und 100°C, bei Anwesenheit eines sauren Katalysators, insbesondere einer Lewis-Säure, wie beispielsweise $BF_3$-Aetherat durchgeführt.

Die Addition von $H_2S$ wird nach der in J.Chem. Soc. 1948 (3), 1894 (London) beschriebenen Methode vorgenommen.

Die erfindungsgemässen Verbindungen stellen flüssige bis viskose, in Schmiermitteln sehr gut lösliche Produkte dar. Sie sind hervorragend als veredelnde Zusätze zu Schmiermitteln geeignet, da sie sowohl die Hochdruckals auch die Antiabnutzungseigenschaften überraschend gut verbessern. Neben dieser ausgezeichneten Wirksamkeit zeigen die erfindungsgemässen Verbindungen auch sehr gute Korrosionsschutzeigenschaften.

Die Verbindungen der Formel I wirken schon in sehr geringen Mengen als Hochdruck-Zusätze in Schmiermitteln. So zeigen mineralische und synthetische Schmieröle, sowie deren Gemische, welche mit 0,01 bis 5 Gew.-%, bezogen auf das Schmiermittel, und vorzugsweise 0,1 bis 3% einer Verbindung der Formel I ausgestattet sind, ausgezeichnete Hochdruck-Schmiereigenschaften, welche durch stark reduzierte Abnutzungserscheinungen der zu schmierenden Teile deutlich werden. Die in Frage kommenden Schmiermittel sind dem Fachmann galäufig und z.B. im "Schmiermittel Taschenbuch" (Hüthig Verlag, Heidelberg, 1974) beschrieben.

Das Schmiermittel kann zusätzlich andere Additive enthalten, die zugegeben werden, um die Eigenschaften zu verbessern, wie Antioxidantien, Metallpassivatoren, Rostinhibitoren, Viscositätsindex-Verbesserer/Fliesspunkterniedriger und Dispergiermittel/Detergentien. Die erfindungsgemässen Verbindungen können auch in Kombination mit anderen Extrem-Druck/Antiabnutzungs-Additiven eingesetzt werden.

Beispiele für Antioxidantien sind:

(a) Alkylierte und nicht-alkylierte aromatische Amine und Mischungen davon, z.B.

Dioctylphenylamin, (2,2,3,3-Tetramethyl-butyl)-phenyl-α- und -β-naphthylamine, Phenothiazin, Dioctylphenothiazin, Phenyl-α-naphthylamin, N,N'-Di-sec-butyl-p-phenylendiamin.

(b) Gehinderte Phenole, z.B. 2,6-Di-tert.-butyl-p-cresol, 4,4'-Bis-(2,6-diisopropylphenol), 2,4,6-Triisopropylphenol, 2,2'-Thio-bis-(4-methyl-6-tert.-butylphenol), 4'4'-Methylen-bis-(2,6-di-t-butylphenol), oder Ester der 2-(3,5-Di-tert.-butyl-4-hydroxyphenyl)-propionsäure mit z.B. Hexan-1,6-diol, Thiodiäthylenglykol und Pentaerythrit.

(c) Alkyl-, Aryl oder Alkaryl-phosphite, z.B.:

Trinonylphosphit, Triphenylphosphit, Diphenyldecylphosphit.

(d) Ester von Thiodipropionsäure oder Thiodiessigsäure, z.B.:

Dilaurylthiodipropionat oder Dioctylthiodiacetat.

(e) Salze von Carbamin- und Dithiophosphor-säuren, z.B.:

Antimon-diamyldithiocarbomat, Zink-diamyldithiophosphat.

(f) Kombination von zwei oder mehr Antioxidantien der obigen, z.B.:

ein alkyliertes Amin und ein sterisch gehindertes Phenol.

Beispiele für Metallpassivatoren sind:

(a) Für Kupfer, z.B.:

Benzotriazol, Tetrahydrobenzotriazol, 2-Mercaptobenzotriazol, 2,5-Dimercaptothiadiazol, Salicylidenpropylendiamin, Salze von Salicylaminoguanidin.

(b) Für Blei, z.B.:

Sebacinsäurederivate, Chinizarin, Propylgallat.

(c) Kombination von zwei oder mehr der obigen Additive.

Beispiele für Rost-Inhibitoren sind:

(a) Organische Säuren, ihre Ester, Metallsalze und Anhydride, z.B.:

N-Oleoyl-sarcosin, Sorbitan-mono-oleat, Blei-naphthenat, Dodecenylbernsteinsäure-anhydrid.

(b) Stickstoffhaltige Verbindungen, z.B.:

I. Primäre, sekundäre oder tertiäre aliphatische oder cycloaliphatische Amine und Amin-Salze von organischen und anorganischen Säuren, z.B. öllösliche Alkylammoniumcarboxylate.

II. Heterocyclische Verbindungen z.B. substituierte Imidazoline und Oxazoline.

(c) Phosphorhaltige Verbindungen, z.B.:

Aminsalze von Phosphorsäurepartialestern.

(d) Schwefelhaltige Verbindungen, z.B.:

Barium-dinonylnaphthalin-sulfonate, Calciumpetroleumsulfonate.

(e) Kombinationen von zwei oder mehr der obigen Additive.

Beispiele für Viskositätsindex-Verbesserer sind z.B.:

Polymethacrylate, Vinylpyrrolidon /Methacrylat-Copolymere, Polybutene, Olefin-Copolymere, Styrol/Acrylat-Copolymere.

Beispiele für Stockpunkterniedriger sind z.B.:

Polymethacrylate, alkylierte Naphthalinderivate.

Beispiele für Dispergiermittel/Tenside sind z.B.:

Polybutenylbersteinsäure-imide. Polybutenylphosphonsäurederivate, basische Magnesium-, Calcium-, und Bariumsulfonate und -phenolate.

Beispiele für andere Verschleisschutz-Additive sind z.B.:

Schwefel und/oder Phosphor und/oder Halogen enthaltende Verbindungen, wie geschwefelte vegetabilische Oele, Zinkdialkyldithiophosphonate, Tritolyl-phosphat, chlorierte Paraffine, Alkyl- und Aryldisulfide.

Die Herstellung der erfindungsgemässen Schmiermittelzusätze sowie ihre Verwendung und Wirkung in Schmiermitteln wird in den folgenden Beispielen näher beschrieben.

## A) Herstellungsbeispiele

### Beispiele 1 bis 4

Nach der Arbeitsvorschrift in Org. Synth. 32 (1952), 39 wird nahezu quantitativ aus dem in Kolonne 2 der nachfolgenden Tabelle I aufgeführten Alkylglycidyläther, durch Umsetzung mit KSCN, die in Kolonne 3 angegebene erfindungsgemässe Epithioverbindung erhalten.

**Tabelle I**

| Beispiel | Alkylglycidyläther | Epithioverbindung | Eigenschaften |
|---|---|---|---|
| 1 | 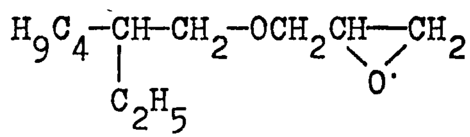 | 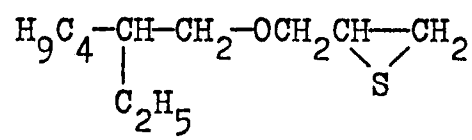 | Kp 91°/0,1 mm<br>$n_D^{22}$ : 1,4699 |
| 2 | 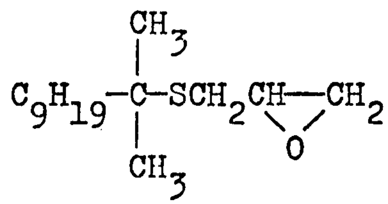 | 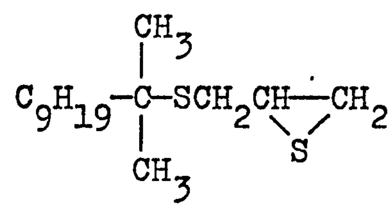 | Kp 119°/0,01 mm<br>$n_D^{22}$ : 1,5049 |
| 3 | 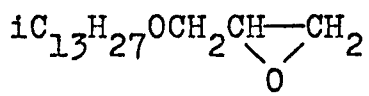 1) | 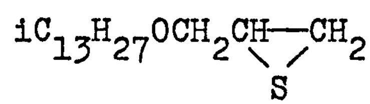 | $n_D^{20}$ : 1,4762 |
| 4 | 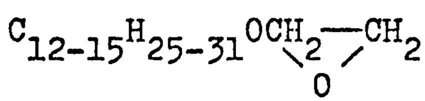 2) | 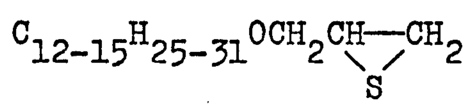 | $n_D^{20}$ : 1,4650 |

1) Ruhrchemie: Iso-tridecanol = Gemisch kurzkettenverzweigter Alkohole (z.B. 2,5,7-Trimethyl-decanol u.a.) als Alkoholkomponente

2) "Dobanol-(25)" (Lieferant: Shell) als Alkoholkomponente.

Beispiel 5

0,1 Mol des Thioglycolsäureesters von 2-Aethylhexanol werden mit 3 Tropfen $BF_3$-Aetherat versetzt und auf dem Wasserbad erwärmt. Man tropft 0,05 Mol Epithio-2-äthylhexyläther zu und rührt eine Stunde bei 60°C. Nach Destillation erhält man das gewünschte Mercaptan der Formel

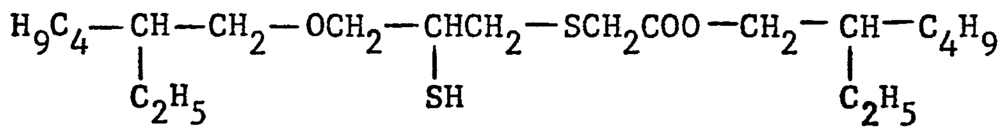

in 85%iger Ausbeute.

Beispiel 6 bis 19

Verfährt man analog dem in Beispiel 3 beschriebenen Verfahren, so erhält man durch Umsetzen entsprechender protonischen Nucleophile (Kolonne 2 der nachfolgenden Tabelle II) mit entsprechenden Epithioverbindungen (Kolonne 3 der Tabelle) weitere erfindungsgemässe Mercaptane (Kolonne 4 der Tabelle).

Tabelle II

| Bei- spiel | Prot. Nucleophil | Epithioverbindung | Mercaptan | Eigenschaften |
|---|---|---|---|---|
| 6 | $H_2S$ | 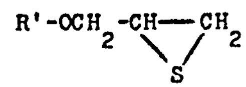 | 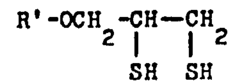 | Kp : 95°C/0,05 mm<br>$n_D^{22}$ : 1,4825 |
| 7 | 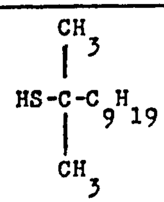 | 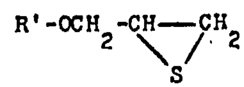 | 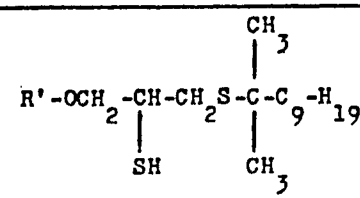 | $n_D^{22}$ : 1,4711 |
| 8 | $H_2N$-n-$C_4H_9$ | 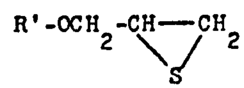 | 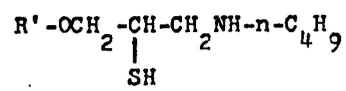 | Kp : 118-122°/<br>95 Torr |
| 9 | HS-R''' | 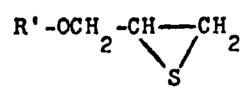 | 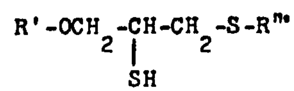 | $n_D^{22}$ : 1,4795 |
| 10 | 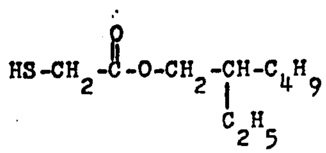 | 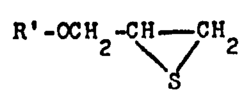 | 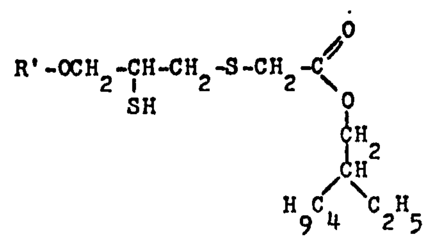 | $n_D^{20}$ : 1. 4898 |

Tabelle II (Forts.)

| Beispiel | Prot. Nucleophil | Epithioverbindung | Mercaptan | Eigenschaften |
|---|---|---|---|---|
| 11 | $HS-CH_2-CH_2OH$ | 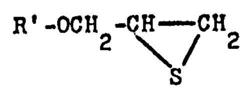 | 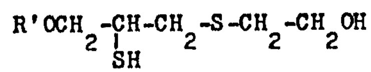 | $n_D^{20}$ : 1,5012 |
| 12 | HS-R''' | 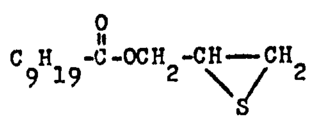 | 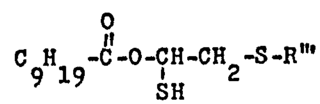 | $n_D^{20}$ : 1,4782 |
| 13 | HS-R''' | 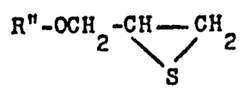 | 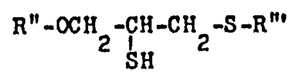 | $n_D^{20}$ : 1,4808 |
| 14 | HS-R''' | 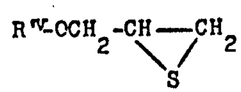 | 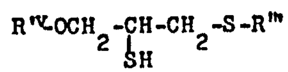 | $n_D^{20}$ : 1,4843 |
| 15 | $H_2S$ | 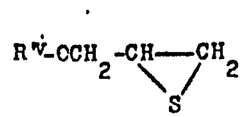 | 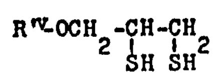 | $n_D^{20}$ : 1,4830 |
| 16 | $H_2S$ | 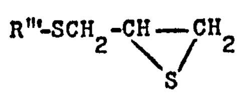 | 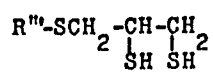 | $n_D^{20}$ : 1,5190 |

Tabelle II (Forts.)

| Beispiel | Prot. Nucleophil | Epithioverbindung | Mercaptan | Eigenschaften |
|---|---|---|---|---|
| 17 | HS-R''' | 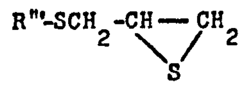 | 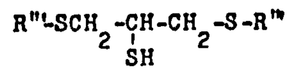 | $n_D^{20}$ : 1,4991 |
| 18 | 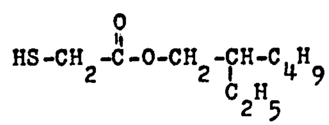 | 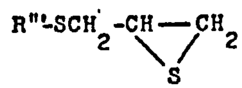 | 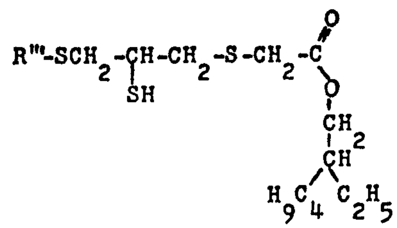 | $n_D^{20}$ : 1,5032 |
| 19 | $HS-CH_2-CH_2-OH$ | 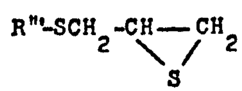 | 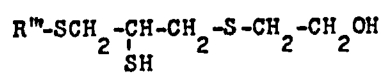 | $n_D^{20}$ : 1,5240 |

R' = 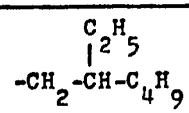

R'' = Gemisch von Alkylgruppen mit einem C-Atomen-Gehalt von 12-15 aus $C_{12-15}H_{25-31}OH$ "Dobanol-(25)" (Shell)

R''' = 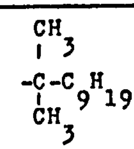

R$^{IV}$ = Gemisch von isomeren verzweigtkettigen $C_{13}$-Alkylgruppen aus Iso-tridecanol (Ruhrchemie)

B) Anwendungsbeispiele

Mit dem Shell-Vierkugel-Apparat wurden folgende Werte bestimmt: (Tentavie method IP 239/69; Extreme pressure and near lubricant test for oils and greases, form ball-machine).

1.) W.L. = weld load. (Schweisslast). Das ist die Last, bei der die 4 Kugeln innerhalb von 10 Sekunden zusammenschweissen.

2.) "Scar diameter" in mm, das ist der mittlere Verschleissdurchmesser nach einer Belastung von 70 kg während 1 Stunde.

3.) In allen Fällen wurde auch die korrosive Wirkung im Kupferstreifentest (Cu-St) geprüft (Beurteilungsskala reicht von la bis 4b).

Als Basisöl wurde Catenex 41 (Handelsbezeichnung der Firma Shell) verwendet. Die Ergebnisse sind in nachfolgender Tabelle III angegeben.

Tabelle III

| Additiv | Konzen-tration | Weld load (kg) | Scar diameter (mm) | Cu-St. |
|---|---|---|---|---|
| - | - | 160 | 2,42 | - |
| Beispiel 1 | 1% | > 200 | 0,6 | 1a |
| Beispiel 2 | 1% | > 200 | 0,8 | 1a |
| Beispiel 3 | 1% | > 200 | 0,5 | 1a |
| Beispiel 4 | 1% | > 200 | 0,5 | 1a |
| Beispiel 5 | 1% | > 200 | 0,6 | 1a |
| Beispiel 7 | 1% | > 200 | 0,5 | 1b |
| Beispiel 9 | 1% | > 180 | 0,4 | 1b |
| Beispiel 10 | 1% | > 200 | 0,3 | 1a |
| Beispiel 11 | 1% | > 200 | 0,6 | 1a |
| Beispiel 12 | 1% | > 180 | 0,5 | 1a |
| Beispiel 13 | 1% | > 180 | 0,4 | 1a |
| Beispiel 14 | 1% | > 180 | 0,5 | 1a |
| Beispiel 15 | 1% | > 200 | 0,7 | 1a |
| Beispiel 16 | 1% | > 200 | 1,1 | 2b |
| Beispiel 17 | 1% | > 180 | > 1,2 | 1a |
| Beispiel 18 | 1% | > 200 | 0,7 | 2c |
| Beispiel 19 | 1% | > 200 | 0,7 | 2e |

Patentansprüche

1. Epithioverbindungen oder Mercaptane der Formel I

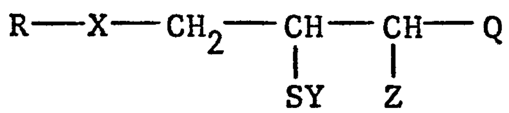

(I),

worin X ein Sauerstoff- oder Schwefelatom, die Gruppen $-CH_2-$, $-CH(R_1)-$, -COO-, -NH- oder $-NR_1-$ ist, Y und Z jeweils Wasserstoff oder zusammen eine direkte Bindung, Q, wenn Y und Z zusammen eine direkte Bindung darstellen, Wasserstoff ist oder Q die Gruppen -OH, -SH, $-OR_1$, $-SR_1$, $-OOCR_1$, $-S-(CH_2)_n-OH$, $-S-(CH_2)_n-COOR_1$, $-NHR_1$ oder $-N(R_1)_2$, n die Zahlen 1 bis 10, R und $R_1$ jeweils unabhängig voneinander einen gegebenenfalls durch Sauerstoff oder Schwefel unterbrochenen cyclischen oder acyclischen Kohlenwasserstoffrest aliphatischen oder aromatischen Charakters mit 4-30 Kohlenstoffatomen bedeuten mit der Bedingung, dass die Summe der C-Atome in den Gruppen R und $R_1$ 8-30 Kohlenstoffatome beträgt.

2. Epithioverbindungen oder Mercaptane gemäss Anspruch 1 der Formel I, worin X ein Sauerstoff- oder Schwefelatom oder die Gruppe -COO- ist, Y und Z die in Anspruch 1 angegebene Bedeutung haben, Q wenn Y und Z zusammen eine direkte Bindung darstellen, Wasserstoff ist oder Q die Gruppen -SH, $-SR_1$, $-S-(CH_2)_2-OH$, $-S-CH_2-COOR_1$, $-NHR_1$ bedeutet und R und $R_1$ jeweils unabhängig voneinander verzweigtes oder unverzweigtes $C_4$-$C_{22}$ Alkyl sind, mit der Bedingung, dass die Summe der C-Atome in den Gruppen R und $R_1$ 8-30 Kohlenstoffatome beträgt.

3. Epithioverbindungen oder Mercaptane gemäss Anspruch 1 der Formel I, worin X ein Sauerstoffatom oder die Gruppe -COO- ist, Y und Z die in Anspruch 1 angegebene Bedeutung haben, Q, wenn Y und Z zusammen eine direkte Bindung darstellen, Wasserstoff ist oder Q die Gruppen -SH, $-SR_1$, $-S-CH_2-COOR_1$, $-NHR_1$ ist und R und $R_1$ jeweils unabhängig voneinander verzweigtes $C_8$-$C_{22}$ Alkyl bedeuten, mit der Bedingung, dass die Summe der C-Atome in den Gruppen R und $R_1$ 8-20 Kohlenstoffatome beträgt.

4. Epithioverbindungen gemäss Anspruch 1 der Formel II

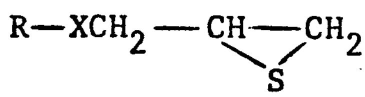 (II),

worin R verzweigtes $C_8$-$C_{18}$ Alkyl und X ein Sauerstoffatom oder die Gruppe -COO- bedeuten.

5. Mercaptane gemäss Anspruch 1 der Formel III

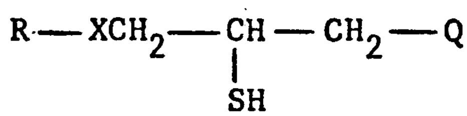 (III),

worin X ein Sauerstoffatom oder die Gruppe -COO- ist, Q die Gruppen $-SR_1$ oder $-S-CH_2-COOR_1$ bedeutet und R und $R_1$ verzweigtes $C_8$-$C_{18}$ Alkyl sind mit der Bedingung, dass die Summe der C-Atome in den Gruppen R und $R_1$ 8-20 Kohlenstoffatome beträgt.

6. Mercaptane gemäss Anspruch 1 der Formel I, dadurch gekennzeichnet, dass R und $R_1$ $C_8$-$C_{22}$ Alkyl und insbesondere verzweigtes Alkyl bedeuten.

7. Mercaptane gemäss Anspruch 1 der Formel I, dadurch gekennzeichnet, dass R und $R_1$ $C_8$-$C_{18}$ Alkyl und insbesondere verzweigtes Alkyl bedeuten.

8. Epithioverbindungen gemäss Anspruch 1 der Formel

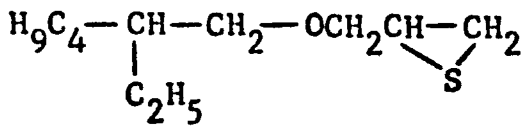

9. Epithioverbindung gemäss Anspruch 1 der Formel

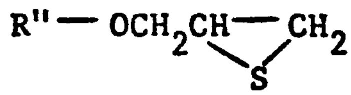

worin R'' ein Gemisch von Alkylgruppen mit einem C-Atomen-Gehalt von 12-15 bedeutet.

10. Mercaptan gemäss Anspruch 1 der Formel

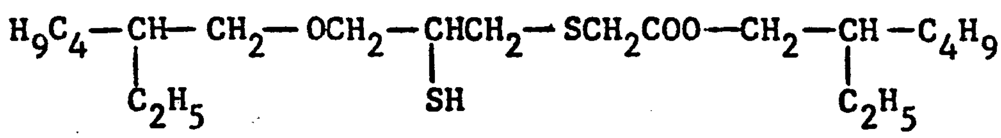

11. Mercaptan gemäss Anspruch 1 der Formel

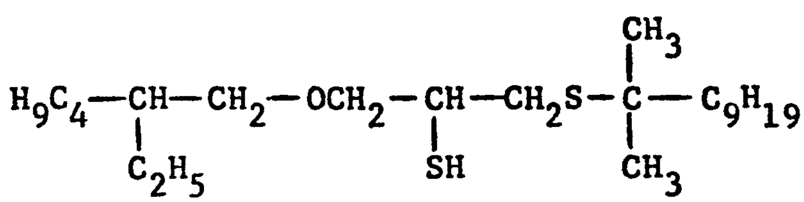

12. Stoffzusammensetzung, enthaltend mineralische und/oder synthetische Schmiermittel und eine wirksame Menge einer Verbindung der Formel I gemäss Anspruch 1.

13. Verwendung von Verbindungen der Formel I, gemäss Anspruch 1 als stabilisierenden Zusatz zu Schmiermitteln.